# EUROPEAN PATENT APPLICATION

(11) **EP 0 616 030 A1**
(43) Date of publication of application: **21.09.1994**
(21) Application number: 94200148.8
(22) Date of filing: 27.01.1994
(51) Int. Cl.: C12N 1/04, C12N 1/18, A21D 8/04

(54) **Instant dry yeast**

(30) Priority: 27.01.1993 EP 93200221
(71) Applicant: GIST-BROCADES N.V., NL-2600 MA Delft (NL)
(72) Inventor: Wegman, Roelof Arie, NL-3291 LD Strijen (NL); Langejan, Arend, NL-2614 GJ Delft (NL)
(74) Representative: Matulewicz, Emil Rudolf Antonius, Dr.

(57) **Abstract**

Instant dry yeast is improved by the addition of a rehydration controlling agent.

## Description

The present invention relates to an instant dry yeast composition, the production thereof and its use in bakery products and beverages.

The manufacture of yeast starts with a small sample of a pure culture. This sample is used to inoculate the first of a series of fermentations in fermentors of successively increasing size. The first few are mildly aerated batch fermentations. Only the last two (or sometimes three) stages are performed using full aeration and incremental feeding of molasses. These fed-batch fermentations are carried out in fermentors having a volume of 100 m³ or more. Fermentation time is typically in the range of 12-20 hours, in which some 10,000-30,000 kg of compressed yeast is produced.

Further processing includes separating the yeast from the broth by centrifugation and washing which results in yeast cream (17-23% (w/w) dry matter content).

The yeast cream may be processed into compressed yeast (27-33% (w/w) dry matter content) which is either sold as such or extruded and dried to produce active dry yeast (ADY) or instant dry yeast (IDY) with moisture contents of 6-8% (w/w) and 2-8% (w/w), respectively.

In the case of ADY, drying usually takes place in belt or rotolouvre (drum) dryers. For IDY production fluidized-bed drying is commonly used. Drying of the yeast to a level of about 20% water content involves only the evaporation of free water. Further reducing of the moisture content involves the removal of a portion of the bound water from the yeast which may cause damage to the yeast cell membrane. In US patent 3,843,800 and US 4,248,420 wetting agents such as esters of saturated fatty acids of glycerol and/or fatty acid esters of propylene glycol are added to preserve the desired high direct leavening activity of the yeast during the drying step.

Dry yeast loses part of its leavening activity during the drying process as well as during the rehydration procedure. Dry yeasts are still commonly used in the bakery trade because of their extended stability and because refrigeration is unnecessary. Dry yeasts are used in wine making to obtain a fast and reproducible fermentation thereby minimizing the risk of failure of the natural fermentation. Moreover, the yeast is immediately available throughout the year.

Instant dry yeast (IDY) is the latest type of baker's yeast, introduced in the early 1970's (see for example US patent 3,843,800) a few years later followed by instant dry wine yeast (IWY), which can be seen as a special form of instant dry yeast. To obtain a high quality IDY, compressed yeast of relatively high protein content (42-60% (w/w)) must be dried in a quick-drying process. The leavening activity of IDY under conditions of application is about 75-85% that of compressed yeast; the shelf life in a vacuum-sealed package is comparable to that of ADY.

IDY is presented typically in the form of very small rods that are highly porous and easy to rehydrate. On the one hand, this allows immediate use, without prior rehydration. On the other hand, the high porosity gives easy access to water and oxygen (from air), which results in a rather rapid loss of activity upon exposure to atmospheric conditions. For satisfactory results, IDY should be used within 3-5 days of opening the package. Moreover, the high porosity of IDY makes it sensitive to extreme rehydration conditions.

IDY usually has a moisture content of 2-8% (w/w) and a protein content between 42 and 60% (w/w) on a dry matter basis.

As with ADY, some manufacturers add antioxidants (e.g. BHA) to their product for improved stability. Ascorbic acid may be added to IDY products to improve stability.

A problem encountered with ADY and IDY is the leakage of yeast solids from the cells upon rehydration. This results in a loss of gassing power or a loss of capacity to produce ethanol. The various methods of adding yeast and mixing dough differ from country to country. Although for the more porous IDY the dry yeast shoud be mixed with flour before water is added, it often happens that the dry yeast is suspended in water together with other soluble additions before flour is added. Additives such as sugar, calciumpropionate and salt affect yeast performance, as does the water temperature. In countries with warm climates or where bakers use high speed mixers with extra heat input, the water is cooled, for example by adding ice, to obtain proper dough temperatures after mixing. Under these conditions instant dry yeast comes in direct contact with the chilled water, thus reducing the yeast performance substantially. In US-A-4,764,472 this problem is partly solved by the incorporation of 0.1 to 2% by weight of locust bean gum, gum ghatti and mixtures thereof, which prevents a loss of activity when water of about 20°C is added. However, in practice, water of 15°C or less, even sometimes a water/ice mixture, is used and at such temperatures activity after rehydration is extremely low.

We have now surprisingly found that when 1.1 to 5% (w/w, dry weight) of a rehydration controlling agent is incorporated in the dry yeast, the yeast is much better protected against the loss of activity due to rehydration of the dry yeast at low temperatures. The present invention therefore provides a dry yeast composition having a moisture content of less than 8% (w/w), preferably 3 to 6% (w/w) and which comprises 1.1 to 5% (w/w) of a rehydration controlling agent. The rehydration controlling agent has preferably a moisture content of less than 10%. When added these agents are often suspended in water to improve the distribution in the yeast.

The rehydration controlling agent is responsible for a controlled rehydration (wetting) of IDY particles and the individual yeast cells. This rehydration controlling agent functions as an extra barrier for water penetration into the cells.

Preferably 1.5 to 3% by weight and more preferably 2 to 3% by weight of a rehydration controlling agent is used. Preferably the rehydration agent comprises at least 50% (w/w) of an emulsifier, for example 55%, 60%, 65%, 70% or 75% (w/w).

Typical examples of rehydration controlling agents are:
- esters of fatty acids such as fatty acid esters of sorbitan, e.g. sorbitan monolaurate, monopalmitate, monostearate or mono-oleate;
- acid esters of mono and/or diglyceride such as citric acid ester or diacetyltartaric acid ester;
- fatty acid esters of glycerol or polyglycerol, e.g. glyceryl monostearate, glyceryl distearate or glyceryl monopalmitate,
- fatty acid esters of propylene glycol e.g. propylene glycol monostearate;
- arabic gum;
- xanthan gum;
- yeast extract;
- CMC (sodium carboxy methyl cellulose)
- or mixtures of two or more of the above mentioned compounds.

We have found that the addition of the rehydration controlling agents according to the invention substantially protects the dry yeast against a loss of activity when water of 15°C, even 10°C or less is added to the yeast.

The rehydration controlling agent is added before the final drying step when the yeast is still wet. The yeast is thus protected during this drying step, thereby preserving the desired high leavening activity of the yeast. When the yeast is mixed in the flour, gassing power under standard conditions is unaffected.

Surprisingly, when the yeast was rehydrated in water, the performance of the instant yeast improves substantially. The greatest improvement is attained at the lowest temperature.

The present invention further provides a method for preparing a dry yeast composition which comprises drying yeast in the presence of 1.1 to 5% (w/w) of a rehydration controlling agent. The present invention additionally provides a method of preparing bread which comprises incorporating into a dough a composition as claimed in any one of claims 1 to 7 and baking the dough.

It will be appreciated that the dough will contain besides the yeast, ingredients generally used in dough preparation.

Surprisingly it has been found that in a dough prepared with the yeast of the present invention the rate of the gas production is increased and the bread volume of the baked product increased substantially.

The amount of rehydration controlling agent present in the yeast may be varied according to different application methods.

All publications and patent applications cited in this specification are herein incorporated by reference as if each individual publication or patent application was specifically and individually indicated to be incorparated.

### Performance of the yeasts prepared according to the present invention.

a. This test was carried out under optimal instant yeast conditions.
   300 mg of instant dry yeast was mixed with 62.5 g of flour. After addition of 34.4 ml of a solution containing 1.25 g of NaCl, the mass is mixed for 6 minutes at 28°C into a dough and placed in a water bath at 28°C. The volume of gas produced within the period from 10 to 175 minutes after the start of mixing was determined in ml at 28°C and 760 mm Hg.
   b1. Identical to (a) except the yeast was wetted as a monolayer of particles on a water surface by means of a small funnel in an erlenmeyer-flask at 35°C, thus allowing every particle to come directly into contact with water. The test was therefore carried out under maximum rehydration conditions.
   b2. Identical to (b1) with water temperature of 20°C.
   b3. Identical to (b1) with water temperature of 10°C.

### Example 1

### Experiment 1:

The tests (Examples 1 to 5) were carried out using fresh baker's yeast, a normal cultivation of strain 210 Ng, CBS 406.87. This product has a moisture content of approximately 68% and a protein content of approximately 50% on dry weight. This block yeast was compresssed to reduce extracellular moisture content to 66%.

This yeast was crumbled and mixed with 0.25% of the rehydration controlling agent Span-60™ (sorbitan monostearate) (on basis of total dry weight) using the following procedure:
The Span-60™ was melted at 65°C and suspended in water at 60°C using a high sheer laboratory mixer. This emulsion was added to compressed baker's yeast up to a content of 0.25% on total dry weight.

The combination was then extruded three times through a screen with holes of 0.8 mm to form rod-shaped particles, containing the rehydration controlling agent properly distributed.

The product from this exercise was pressed finally through a screen with holes of 0.8 mm to form again rod-shaped particles.

The drying of this product was performed in a laboratory scale fluid bed-dryer, consisting of a conical glass tube built on an air supply system, fenced by an appropriate screen to create a calm fluidisation pattern. The air supply system consisted of a two way control valve, leading air partly through an electrical heating section. By means of this control valve a constant outlet air temperature of 39°C was maintained during the drying. The airflow was set at a superficial speed of 1.6 m/s at the bottom of the conus.

The airflow was stopped as soon as the inlet-air temperature had reached a temperature of 45°C.

The product was collected and packed in sachets under vacuum for analysing.

### Experiment 2

As described in test 1, but with the Span-60™ constituting 0.50% of the total dry matter.

### Experiment 3

As described in test 1, but with the Span-60™ constituting 0.75% of the total dry matter.

### Experiment 4

As described in test 1, but with the Span-60™ constituting 1.0% of the total dry matter.

### Experiment 5

As described in test 1, but with the Span-60™ constituting 1.50% of the total dry matter.

### Experiment 6

As described in test 1, but with the Span-60™ constituting 2.0% of the total dry matter.

### Experiment 7

As described in test 1, but with the Span-60™ constituting 3.0% of the total dry matter.
Results in gassing power of Example 1:

| Addition | Result test a (ml) | Result test b1 (ml) | Result test b2 (ml) | Result test b3 (ml) |
|---|---|---|---|---|
| Span-60™ 0.25% | 283 | 173 | 83 | 29 |
| Span-60™ 0.5% | 290 | 207 | 130 | 69 |
| Span-60™ 0.75% | 290 | 223 | 159 | 85 |
| Span-60™ 1.0% | 300 | 227 | 170 | 88 |
| Span-60™ 1.5% | 304 | 246 | 197 | 122 |
| Span-60™ 2.0% | 303 | 249 | 189 | 123 |
| Span-60™ 3.0% | 298 | 248 | 201 | 129 |

### Example 2

The experiment was carried out in a manner identical to Example 1, but with a commercially available citric acid ester of glycerine monostearate (CGM) as rehydration controlling agent.
Results in gassing power of Example 2:

| Addition | Result test a (ml) | Result test b1 (ml) | Result test b2 (ml) | Result test b3 (ml) |
|---|---|---|---|---|
| CGM 0.25% | 288 | 174 | 96 | 37 |
| CGM 0.5% | 290 | 193 | 128 | 54 |
| CGM 0.75% | 297 | 215 | 146 | 71 |
| CGM 1.0% | 300 | 225 | 165 | 93 |
| CGM 1.5% | 303 | 241 | 181 | 98 |
| CGM 2.0% | 307 | 233 | 189 | 109 |
| CGM 3.0% | 310 | 249 | 199 | 121 |

### Example 3

Identical to experiment 1 of Example 1 except for the concentration of rehydration controlling agent being Span-60™ at a final concentration of 1.0% and an extra addition of yeast extract powder to a final concentration of 2.0%.

### Example 4

Identical to experiment 1 of Example 1 except for the concentration of rehydration controlling agent being Span-60™ at a final concentration of 1.0% and xanthane gum solution to a final concentration of 1.4%.

### Example 5

Identical to experiment 1 of Example 1 except for the concentration of rehydration controlling agent being Span-60™ at a final concentration of 1.0% and sodium carboxy methyl cellulose to a final concentration of 1.0%.
Results in gassing power of Example 3 to 5.

| Addition | Result test a (ml) | Result test b1 (ml) | Result test b2 (ml) | Result test b3 (ml) |
|---|---|---|---|---|
| Span-60™ 1.0% + low salt yeast extract 2.0% (d.m.) | 298 | 244 | 229 | 133 |
| Span-60™ 1.0% + xanthane gum 1.4% (d.m.) | 308 | 282 | 246 | 157 |
| Span-60™ 1.0% + sodium carboxy methyl cellulose 1.0% (d.m.) | 299 | 261 | 213 | 151 |

### Example 6

Identical to experiment 4 of Example 1 except for the strain applied being 227 Ng, CBS 155.91 (deposited on March 5, 1991).

### Example 7

Identical to Example 6, except for the choice and concentration of rehydration controlling agent being CGM at a final concentration of 2.5%.

### Example 8

Identical to Example 6, except for the choice of rehydration controlling agent being a combination of 1.0% Span-60™ and 1.0% sodium carboxy methyl cellulose dissolved as a 5% solution in water before mixing with the Span-60™ emulsion.
Results Example 6, 7, and 8.

| Addition | Result test a (ml) | Result test b3 (ml) |
|---|---|---|
| Span-60™ 1.0% | 325 | 87 |
| CGM 2.5% | 324 | 159 |
| Span-60™ 1.0% + sodium carboxy methyl cellulose 1.0% (d.m.) | 319 | 146 |

### Example 9

Identical to experiment 4 of Example 1, except for the strain applied being 237 Ng, CBS 158.86 (deposited on March 25, 1986) a sugar resistant type of strain.

Contrary to Examples 1 to 8 the gas performance tests with the samples of Example 9, 10 and 11 were carried out applying an extra addition of 10% sugar on flour weight.

### Example 10

Identical to Example 9, except for the choice and concentration of rehydration controlling agent being CGM at a final concentration of 2.5%.

### Example 11

Identical to Example 9, except for the choice of rehydration controlling agent being a combination of Span-60™ and sodium carboxy methyl cellulose as in Example 8.
Results Example 9, 10 and 11

| Addition | Result test a (ml) | Result test b3 (ml) |
|---|---|---|
| Span-60™ 1.0% | 270 | 59 |
| CGM 2.5% | 268 | 89 |
| Span-60™ 1.0% + sodium carboxy methyl cellulose 1.0% (d.m.) | 271 | 92 |

### Example 12

In one type of baking trial producing French batard type of bread (method I) 3000 g wheat flour having a temperature of 20°C, 1.75% salt, 1% Unipan Plus® (bread improver, Gist-brocades), and 0.45% instant dry yeast of 227 Ng strain (CBS 155.91), containing varying contents of rehydration controlling agent being CGM, are mixed by hand in the bowl of a Phebus mixer. Afterwards 56% water having a temperature of 32°C is added and kneading starts (2 minutes at speed 1 and 18 minutes at speed 2). The dough temperature is 27°C. The dough is given a first proof of 15 minutes in a proofing cabinet at 30°C and 85% RH. Afterwards the dough is divided into 12 pieces of 350 g. These pieces are moulded and given an intermediate proof of 15 minutes at 30°C and 85% RH. After this stage the pieces are again moulded, shaped, and given a final proof of 110 minutes at 30°C and 85% RH. Afterwards the fully proofed doughs are brought into the oven and baked at 240°C for 25 minutes.

In a second type of baking trial producing French batard type of bread (method II), 3000 g wheat flour having a temperature of 42°C, 1.75% salt, 1% Unipan Plus® (bread improver, Gist-brocades) are introduced in the bowl of the Phebus mixer. 0.45% Instant dry yeast of 227 Ng strain, containing varying contents of rehydration controlling agent being CGM, is spread over the surface of this mixture. Afterwards 56% water having a temperature of 4°C is poured out over the surface and mixing starts with 2 minutes at speed 1 and 28 minutes at speed 2 to reach the dough temperature of 27%. Dough handling, fermentation and baking procedure is the same as described above, only the final proof time is varying. Final proof time is determined by putting 45 g of dough (after first and second proof) in a standardized measuring cylinder and let it rise to a fixed height of 10 cm.

In both methods, after cooling down to room temperature, loaf volumes are obtained by the rapeseed displacement method. Results are shown in the Table hereinbelow. Loaf volumes are an average of volume measurements of 4 loaves of bread.

From this Table it is clear that in method I, using flour of 20°C and water of 32°C, an increase in loaf volume is seen after introducing instant yeast containing a higher rehydration controlling agent.

This result corresponds to the gassing results shown in Example 2.

Breadmaking according to method II leads to shorter final proof times and somewhat higher loaf volumes in those cases where instant yeast is used having a higher rehydration controlling agent.

| Loaf volumes of Example 12 | | | |
|---|---|---|---|
| addition | Method I | Method II | |
| | Loaf volume (ml) | Final proof time (min.) | Loaf volume (ml) |
| CGM 1.0% | 1460 | 160 | 1336 |
| CGM 1.5% | 1472 | 150 | 1393 |
| CGM 2.0% | 1514 | 135 | 1419 |
| CGM 3.0% | 1563 | 135 | 1377 |

As a consequence of the shorter final proof times the overall bread quality is improved: a better break and shred of the crust and a finer texture of the crumb.

## Claims

1. A dry yeast composition having a moisture content of less than 8% (w/w) which comprises 1.1 to 5% (w/w) of a rehydration controlling agent.

2. A composition according to claim 1 wherein the rehydration controlling agent comprises at least one of
- an ester of a fatty acid
- an acid ester of a mono and/or a diglyceride
- arabic gum;
- xanthan gum;
- yeast extract or
- sodium carboxy methyl cellulose (CMC);

3. A composition according to claim 1 or 2 wherein the ester of fatty acid is a sorbitan ester, a glycerol ester, a polyglycerol ester of a propylene glycol ester.

4. A composition according to claim 3 wherein the ester of a fatty acid is sorbitan monolaurate, sorbitan monopalmitate, sorbitan monostearate or sorbitan monooleate, glyceryl monostearate, glyceryl distearate, glyceryl monopalmitate or propylene glycol monostearate.

5. A composition according to claim 2 wherein the acid ester of the mono and/or diglyceride is a citric acid ester or a diacetyltartaric acid ester.

6. A composition according to any one of the preceding claims which comprises from 1.5 to 3% (w/w), preferably 2 to 3% (w/w) of the rehydration controlling agent.

7. A composition according to any one of the preceding claims wherein the rehydration controlling agent comprises at least 50% w/w emulsifier.

8. A method for preparing a dry yeast composition which comprises drying yeast in the presence of 1.1 to 5% (w/w) of a rehydration controlling agent.

9. A method according to claim 8 wherein the rehydration controlling agent is as defined in any one of claims 2 to 7.

10. A method of preventing loss of yeast activity during rehydration which comprises incorporating a rehydration controlling agent into the yeast during the drying thereof.

11. A method of preparing bread which comprises incorporating into a dough a composition as claimed in any one of claims 1 to 7 or 11 and baking the dough.

12. Use of a rehydration controlling agent as defined in any one of claims 2 to 7 for protecting the yeast during hydration to produce yeast with a moisture content of less than 8% (w/w).

13. Use of a composition according to any one of claim 2 to 7 for the preparation of a dough whereby water of 0 to 15°C is used.
